# EUROPÄISCHE PATENTSCHRIFT

(11) **EP 3 727 278 B1**
(45) Veröffentlichungstag und Bekanntmachung des Hinweises auf die Patenterteilung: **22.07.2026**
(21) Anmeldenummer: 17822313.7
(22) Anmeldetag: 22.12.2017
(51) Int. Cl.: A61K 8/06, A61K 8/34, A61Q 5/02, A61Q 15/00, A61Q 17/04, A61Q 19/00, A61Q 19/10

(54) **KOSMETISCHE EMULSION, UMFASSEND 1,2-DECANDIOL**
COSMETIC EMULSION COMPRISING 1,2-DECANEDIOL
ÉMULSION COSMÉTIQUE COMPRENANT DU 1,2-DÉCANEDIOL

(43) Veröffentlichungstag der Anmeldung: 28.10.2020
(73) Patentinhaber: Symrise AG, 37603 Holzminden (DE)
(72) Erfinder: SCHMAUS, Gerhard, 37671 Höxter-Bosseborn (DE); EBBECKE, Jan Peter, 37620 Halle (DE); LANGE, Sabine, 37603 Holzminden (DE)
(74) Vertreter: Eisenführ Speiser
(86) Internationale Anmeldenummer: PCT/EP2017/084375
(87) Internationale Veröffentlichungsnummer: WO 2019/120564

(56) Entgegenhaltungen:
- EP-A1- 2 243 462
- EP-A1- 2 589 291
- EP-A1- 2 745 831
- EP-A1- 3 045 161
- EP-A2- 1 872 770
- CN-A- 104 027 270
- DE-A1- 10 308 565
- BOSCIA CLEAR COMPLEXION: "Moisturizer with Botanical Blast", GNPD; MINTEL, 1 December 2010 (2010-12-01), XP002743241

## Beschreibung

Die Haut ist ein wichtiges, aber zugleich auch empfindliches Organ, deren Pflege für die Gesundheit sowie für das körperliche und geistige Wohlbefinden unentbehrlich ist. Aus diesem Grund wurden bereits zahlreiche Hautpflegeprodukte und Kosmetika entwickelt, die zum Beispiel als Cremes, Lotionen, Öle, Sprays oder Gele erhältlich sind und verschiedene Hautpflege-Wirkstoffe enthalten.

Häufig liegen Hautpflegeprodukte und Kosmetika in Form von Emulsionen vor. Emulsionen sind heterogene Systeme aus mindestens zwei normalerweise nicht mischbaren Flüssigkeiten (Phasen), wobei eine Flüssigkeit (Phase) kleine Tröpfchen bildet, die verteilt in der anderen Flüssigkeit (Phase) vorliegen. Die Phase, die Tröpfchen bildet, wird als disperse Phase bezeichnet. Die Phase, in der die Tröpfchen vorliegen, wird als kontinuierliche Phase bezeichnet.

Die in der Kosmetikindustrie wichtigsten Emulsionen sind Öl-in-Wasser (O/W)- und Wasser-in-Öl (W/O)-Emulsionen. Diese Emulsionen werden auch als Einfach-Emulsionen bezeichnet. O/W-Emulsionen weisen als kontinuierliche Phase Wasser und als disperse Phase ein Öl auf. W/O-Emulsionen weisen als kontinuierliche Phase ein Öl und als disperse Phase Wasser auf. Der Grundcharakter einer O/W-Emulsion wird durch das Wasser geprägt. Der Grundcharakter einer W/O-Emulsion wird durch das Öl geprägt.

Unter einem Öl wird im Rahmen dieser Erfindung eine organische Flüssigkeit verstanden, die sich unter normalen Umständen nicht mit Wasser mischen lässt. In kosmetischen Emulsionen wird in der Regel deionisiertes Wasser (auch als demineralisiertes Wasser, destilliertes Wasser oder Reinstwasser bekannt) verwendet.

Eine wichtige Eigenschaft einer kosmetischen Emulsion ist, neben der kosmetischen Wirksamkeit, ihre physikalische Stabilität. Denn auch eine homogen und stabil erscheinende Emulsion kann sich mit zunehmender Lagerdauer verändern. Diese Veränderungen können sowohl unbemerkt sein oder aber zu einer deutlich sichtbaren Phasentrennung, einem sogenannten "Aufbrechen" oder "Brechen" der Emulsion mit deutlich sichtbaren Wasser- bzw. Ölabscheidungen führen. Diese Veränderungsprozesse können die emulsionscharakteristischen Eigenschaften so stark beeinflussen, dass die Emulsion den Erwartungen, z.B. an ein kosmetisches Produkt, nicht mehr entspricht und unbrauchbar wird.

Ohne an eine bestimmte Theorie gebunden zu sein, liegt die unerwünschte Phasentrennung in der thermodynamischen Instabilität einer Emulsion begründet, die eine Folge einer unter normalen Umständen hohen Grenzflächenspannung zwischen den Phasen ist, welche wiederum eine Folge der unterschiedlichen intramolekularen Anziehungskräfte innerhalb der jeweiligen Phasen ist. Emulsionen zeigen daher ein generelles Bestreben nach einer möglichst geringen Grenzfläche zwischen den einzelnen (unter normalem Umständen nicht mischbaren) Phasen und somit nach einer Trennung der Phasen.

Um diesem Bestreben entgegenzuwirken und Emulsionen über einen längeren Zeitraum stabil zu halten, werden Emulsionen üblicherweise Emulgatoren zugesetzt, die die Oberflächen- bzw. Grenzflächenspannung der Emulsionen herabsetzen. Bei Emulgatoren handelt es sich um Moleküle mit einem polaren, hydrophilen (also wasserliebenden) Strukturelement und einem unpolaren lipophilen (also ölliebenden) Strukturelement. Aufgrund ihres amphiphilen Charakters lagern sich die einzelnen Emulgatormoleküle an den Grenzflächen an und reduzieren die Oberflächen- bzw. Grenzflächenspannung. Zusammen mit einem Eintrag von mechanischer Arbeit und ggf. thermischer Energie entsteht so ein fein disperses System, in dem eine Phase fein verteilt in der anderen Phase in Form von Tröpfchen vorliegt.

Es besteht daher generell der Bedarf an Verbindungen, die die Oberflächen- bzw. Grenzflächenspannung einer Emulsion herabsetzen. Insbesondere besteht der Bedarf an Verbindungen, die die Oberflächen- bzw. Grenzflächenspannung einer Emulsion bereits in einer geringen Einsatzmenge stark herabsetzen.

Eine Verbindungsklasse, der oberflächen- bzw. grenzflächenspannungsreduzierende Eigenschaften zugesprochen wird, sind 1,2-Alkandiole.

1,2-Alkandiole werden auch als Benetzungsmittel bezeichnet. Sie reduzieren zwar die Grenzflächenspannung zwischen wässriger Phase und Ölphase, sind aber allein nicht in der Lage stabile Emulsionen zu bilden. Folglich werden 1,2-Alkandiole nach dem HLB-Konzept von Griffin (vgl. Griffin, W. C.: Classification of surface active agents by HLB, J. Soc. Cosmet. Chem. 1, 1949) nicht den emulgierenden Substanzen (Emulgatoren) zugeordnet.

Neben einer oberflächen- bzw. grenzflächenspannungsreduzierenden Eigenschaft werden 1,2-Alkandiolen auch andere positive Eigenschaften, wie zum Beispiel feuchtigkeitsspendende oder antimikrobielle Eigenschaften, zugesprochen.

So sind aus dem Stand der Technik bereits die Verwendung von 1,2-Alkandiolen in kosmetischen Emulsionen sowie kosmetische Emulsionen bekannt, die 1,2-Akandiole umfassen.

Aus EP 2 589 291 ist die Verwendung von 1,2-Decandiol, 1,2-Octandiol, 1,2-Hexandiol und 1,2-Pentandiol, in kosmetisch oder dermatologisch wirksamen Mischungen bekannt.

Aus DE 10 2012 224 158 ist eine kosmetische Emulsion bekannt, die Polyglycerol-3-Methylglucose Distearat, Silica Dimethyl Silylate und Acrylates/C10-C30 Alkylacrylates Crosspolymer enthält.

Aus EP 1 946 742 ist eine kosmetische Wasser-in-Silikonöl-Emulsion bekannt, die 1,2-Decandiol enthält.

Aus EP 1 426 029 A1 ist eine langzeitstabile Emulsion bekannt, die 1,2-Alkandiole umfasst.

EP 2 243 462 A1 offenbart ein Emulgatorsystem, das einen Basisemulgator enthält, einen Konsistenzgeber und/oder Co-Emulgator und ein Benetzmittel mit einem Molekulargewicht zwischen 100 und 250 g/mol und einem HLB-Wert zwischen 6 und 12, ausgewählt aus der Gruppe der Glycerinmonoester mit Fettsauren einer Kettenlänge von n = 6 - 10 Kohlenstoffatomen oder der 1,2-Alkandiole mit einer Kettenlänge n = 5 - 10 Kohlenstoffatomen.

Ein weiteres Problem von kosmetischen Emulsionen ist, dass Emulgatoren zwar generell benötigt werden, um der Emulsion eine gewisse (physikalische) Stabilität zu verleihen, diese Emulgatoren aber in der Regel keine kosmetische Wirkung entfalten. Daher ist es erstrebenswert, die Gesamtemulgatormenge (d.h. die Gesamtmenge aller in der Emulsion vorhandenen Emulgatoren) so gering wie möglich zu halten, ohne dabei aber zugleich die (physikalische) Stabilität der Emulsion zu verringern. Es besteht daher das Bedürfnis nach Mitteln und Wegen, die Gesamtemulgatormenge einer Emulsion zu verringern, ohne dabei zugleich die (physikalische) Stabilität zu verringern. Auch wenn dies auf den ersten Blick widersprüchlich erscheint, ist es besonders erstrebenswert, die Gesamtemulgatormenge einer Emulsion zu verringern und gleichzeitig die (physikalische) Stabilität zu erhöhen.

Es ist daher eine Aufgabe der vorliegenden Erfindung, die Menge von in kosmetischen Emulsionen verwendeten Emulgatoren zu verringern, ohne aber gleichzeitig die (physikalische) Stabilität der Emulsion zu verringern, bestenfalls die (physikalische) Stabilität sogar (trotz der verringerten Gesamtemulgatormenge) zu erhöhen.

Diese Aufgabe und weitere Aufgaben wird/werden gelöst durch eine kosmetische Emulsion, umfassend
eine kontinuierliche Wasserphase,
in der kontinuierlichen Wasserphase verteilt vorliegende Öltröpfchen und
1,2-Decandiol,
wobei die in der kontinuierlichen Wasserphase verteilt vorliegenden Öltröpfchen einen durchschnittlichen volumenbezogenen Durchmesser d_{v 0,5} von 1,0 bis 10,0 µm, bevorzugt 3,0 bis 10,0 µm, weiter bevorzugt 5,0 bis 10,0 µm, aufweisen
   und
wobei die Emulsion ferner einen oder mehrere von 1,2-Decandiol verschiedene/n Emulgator/en umfasst
   und
wobei der eine oder die mehreren von 1,2-Decandiol verschiedene/n Emulgator/en in einer Gesamtmenge von 0,1 bis 5,0 Gew.-%, bevorzugt 0,1 bis 3,0 Gew.-%, weiter bevorzugt 0,5 bis 2,5 Gew.-%, noch weiter bevorzugt 1,0 bis 2,3 Gew.-%, noch weiter bevorzugt 1,0 bis 1,8 Gew.-% am bevorzugtesten 1,6 bis 1,8 Gew.-%, in der Emulsion vorhanden sind, bezogen auf das Gesamtgewicht der Emulsion
   und
wobei 1,2-Decandiol in einer Menge im Bereich von 0,005 bis 0,1 Gew.-%, bevorzugt von 0,005 bis 0,05 Gew.-% oder von 0,05 bis 0,1 Gew.-%, in der Emulsion vorhanden ist, bezogen auf das Gesamtgewicht der Emulsion,
   und
wobei der eine oder die mehreren von 1,2-Decandiol verschiedene/n Emulgator/en ausgewählt ist/sind aus der Gruppe bestehend aus PEG-100 Stearat, Cetearyl Glucosid, Distearyldimoniumchlorid, Palmitamidopropyl-trimoniumchlorid, Glycerylstearatcitrat, Glyceryloleatcitrat, Polyglyceryl-(3)-Methylglucosedistearat, Cetearylalkohol, Kaliumcetylphosphat, Natriumcetylphosphat, Acrylat/C10-C30-Alkylacrylat-Kreuzpolymer, Ammoniumacryloyldimethyltaurat/Beheneth-25 Methacrylat-Kreuzpolymer, Polyglyceryl-4-Caprat, Polyglyceryl-4-Caprylat/Caprat, Cetyl PEG/PPG-10/1 Dimethicon, Polyglyceryl-6 Dioleat, Polyglyceryl-2-Stearat, PEG-30 Dipoly-hydroxystearat, Natriumstearoyllactylat, PEG-40 hydriertes Rizinusöl, hydrierten Palmglyceriden oder einer Mischung davon.

Bei der erfindungsgemäßen kosmetischen Emulsion handelt es sich also um eine O/W-Emulsion mit einer kontinuierlichen Wasserphase und einer dispersen Ölphase.

### 1,2-Decandiol hat die folgende Strukturformel

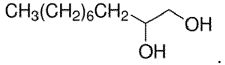

Nach dem HLB-Konzept von Griffin hat 1,2-Decandiol einen HLB-Wert von etwa 3,9. Im Vergleich dazu hat 1,2-Pentandiol einen HLB-Wert von 6,6, 1,2-Hexandiol einen HLB-Wert von 5,8 und 1,2-Octandiol einen HLB-Wert von 4,7.

Im Rahmen der vorliegenden Erfindung wurde der durchschnittliche volumenbezogene Durchmesser der Öltröpfchen bevorzugt mittels des Prinzips der Laserbeugungsspektroskopie bestimmt. Entsprechende Geräte zur Bestimmung des durchschnittlichen volumenbezogenen Durchmessers werden von z.B. Malvern Insruments GmbH und der Malvern Instruments Ltd. vertrieben. Sofern nachfolgend nicht anders angegeben, wird der durchschnittliche volumenbezogene Durchmesser mittels eines Geräts des Typs Malvern "Mastersizer 3000" bestimmt.

d_{v 0,5} XY bedeutet, dass 50% der Öltröpfchen einen volumenbezogenen Durchmesser aufweisen, der kleiner ist als XY. So bedeutet beispielsweise d_{v 0,5} 20 µm, dass 50% der Öltröpfchen einen volumenbezogenen Durchmesser aufweisen, der kleiner ist als 20 µm.

Dementsprechend bedeutet d_{v 0,1} XY, dass 10% der Öltröpfchen einen volumenbezogenen Durchmesser aufweisen, der kleiner ist als XY. So bedeutet beispielsweise d_{v 0,1} 20 µm, dass 10% der Öltröpfchen einen volumenbezogenen Durchmesser aufweisen, der kleiner ist als 20 µm.

d_{v 0,9} XY bedeutet, dass 90% der Öltröpfchen einen volumenbezogenen Durchmesser aufweisen, der kleiner ist als XY. So bedeutet beispielsweise d_{v 0,9} 20 µm, dass 90% der Öltröpfchen einen volumenbezogenen Durchmesser aufweisen, der kleiner ist als 20 µm.

Bevorzugt wird deionisiertes Wasser verwendet. Es kann jedoch in Abhängigkeit der herzustellenden Emulsion auch übliches Leitungswasser verwendet werden. Der Fachmann kann die Art des zu verwendenden Wassers nach seinem Fachwissen auswählen.

Es ist bevorzugt, dass die erfindungsgemäße Emulsion neben 1,2-Decandiol keine weiteren 1,2-Alkandiole enthält.

### ÖLPHASE

Bevorzugt ist die erfindungsgemäße Emulsion ein Zweiphasensystem aus einem kosmetischen Öl in Wasser.

Unter einem kosmetischen Öl wird im Rahmen der vorliegenden Erfindung ein Öl verstanden, das hautverträglich ist und/oder einen positiven kosmetischen Effekt ausübt.

Das in der erfindungsgemäßen kosmetischen Emulsion eingesetzte Öl ist bevorzugt ausgewählt aus der Gruppe bestehend aus
Guerbet-Alkoholen auf Basis von Fettalkoholen mit 6 bis 18, bevorzugt 8 bis 10, Kohlenstoffatomen,
Estern von linearen C₆-C₂₂-Fettsäuren mit geradkettigen oder
verzweigten C₆-C₂₂-Fettalkoholen oder Estern von verzweigten C₆-C₁₃-Carbonsäuren mit geradkettigen oder verzweigten C₆-C₂₂-Fettalkoholen.

Bevorzugt ist das in der erfindungsgemäßen kosmetischen Emulsion eingesetzte Öl ausgewählt aus der Gruppe bestehend aus Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat.

### Weitere bevorzugte Öle sind

Ester von geradkettigen C₆-C₂₂-Fettsäuren mit verzweigten Alkoholen, insbesondere 2-Ethylhexanol,
Ester von C₁₈-C₃₈-Alkylhydroxy-Carbonsäuren mit geradkettigen oder verzweigten C₆-C₂₂-Fettalkoholen, insbesondere Dioctylmalat,
Ester von geradkettigen oder verzweigten Fettsäuren mit mehrwertigen Alkoholen (zum Beispiel Propylenglycol, Dimerdiol oder Trimertriol), Guerbet-Alkoholen, Triglyceriden basierend auf C₆-C₁₀-Fettsäuren und/oder flüssigen Mono-/Di-/Triglyceridgemischen basierend auf C₆-C₁₈-Fettsäuren,
Ester von aromatischen Carbonsäuren, insbesondere Benzoesäure, mit C₆-C₂₂-Fettalkoholen und/oder Guerbet-Alkoholen,
Ester von C₂-C₁₂-Dicarbonsäuren mit geradkettigen oder verzweigten Alkoholen mit 1 bis 22 Kohlenstoffatomen und/oder Polyolen mit 2 bis 10 Kohlenstoffatomen und 2 bis 6 Hydroxylgruppen,
Pflanzenöle,
verzweigte primäre Alkohole,
substitutierte Cyclohexane,
geradkettige und verzweigte C₆-C₂₂-Fettalkoholcarbonate, zum Beispiel Dicaprylylcarbonat (Cetiol^{®} CC),
Guerbet-Carbonate basierend auf Fettalkoholen mit 6 bis 18, bevorzugt 8 bis 10, Kohlenstoffatomen,
Ester von Benzoesäure mit geradkettigen und/oder verzweigten C₆-C₂₂-Alkoholen (z.B. Finsolv^{®} TN),
geradkettige oder verzweigte symmetrische oder asymmetrische Dialkylether mit 6 to 22 Kohlenstoffatomen pro Alkylgruppe, zum Beispiel Dicaprylylether (Cetiol^{®} OE), und/oder Ringöffnungsprodukte von epoxidierten Fettsäureestern mit Polyolen.

Insbesondere geeignet sind Caprylic/Capric Triglycerid (auch als Neutralöl bekannt) und Cetearylethylhexanoat.

Ferner sind Silikonöle (Cyclomethicone, Siliconmethicone usw.) und/oder aliphatische oder naphthenische Kohlenwasserstoffe, zum Beispiel Squalane, Squalene oder Dialkylcyclohexane geeignet. Es ist aber bevorzugt, dass die erfindungsgemäße Emulsion weniger als 25 Gew.-%, bevorzugt weniger als 20 Gew.-%, weiter bevorzugt weniger als 10 Gew.-%, besonders bevorzugt weniger als 5 Gew.-%, am bevorzugtesten 0 Gew.-%, silikonhaltige Öle aufweist, bezogen auf das Gesamtgewicht der Emulsion. Unter einem silikonhaltigen Öl wird im Rahmen der vorliegenden Erfindung ein Öl verstanden, das die Struktureinheit [R¹R²SiO]ₙ aufweist, in der R¹ und R² zum Beispiel organische Reste sind und n eine ganze Zahl ist.

Die Wahl der Komponenten der Ölphase und die massenmäßige Zusammensetzung der Ölphase sind maßgeblich für das Spreitvermögen der kosmetischen Emulsion auf der Haut verantwortlich, was wiederum maßgeblich das Hautgefühl beeinflusst.

Gemäß einer bevorzugten Ausführungsform umfasst die Ölphase Ethylhexylisononanoat, Caprylic/Capric Triglycerid und Prunus Amygdalus Dulcis (Sweet Almond) Öl oder besteht daraus. Es hat sich gezeigt, dass diese Kombination aus niedrig-, mittel- und hochspreitenden Substanzen der Emulsion eine gute Verteilbarkeit auf der Hautoberfläche verleiht und für ein angenehmes Hautgefühl sorgt.

Besonders bevorzugt umfasst die Ölphase Caprylic/Capric Triglycerid und Cetearylethylhexanoat oder besteht daraus. Besonders bevorzugt beträgt das Gewichtsverhältnis von Caprylic/Capric Triglycerid zu Cetearylethylhexanoat 2:1. Besonders bevorzugt umfasst die Ölphase Caprylic/Capric Triglycerid und Cetearylethylhexanoat in einer Gesamtmenge von 1,0 bis 50,0 Gew.-%, bevorzugt 3,0 bis 30,0 Gew.-%, weiter bevorzugt 5,0 bis 20,0 Gew.-%, weiter bevorzugt 8,0 bis 15,0 Gew.-%, am bevorzugtesten 10,0 bis 13,0 Gew.-%, oder besteht daraus, bezogen auf das Gesamtgewicht der Emulsion. Gemäß einer weiteren bevorzugten Ausführungsform umfasst die Ölphase Caprylic/Capric Triglycerid in einer Menge von 2,0 bis 15,0 Gew.-%, bevorzugt 4,0 bis 12,0 Gew.-%, weiter bevorzugt 5,0 bis 10,0 Gew.-%, am bevorzugtesten 7,0 bis 9,0 Gew.-%, und/oder Cetearylethylhexanoat in einer Menge von 1,0 bis 10,0 Gew.-%, bevorzugt 2,0 bis 8,0 Gew.-%, weiter bevorzugt 3,0 bis 5,0 Gew.-%, oder besteht daraus, jeweils bezogen auf das Gesamtgewicht der Emulsion. Gemäß einer besonders bevorzugten Ausführungsform umfasst die Ölphase Caprylic/Capric Triglycerid in einer Menge von 7,0 bis 9,0 Gew.-% und Cetearylethylhexanoat in einer Menge von 3,0 bis 5,0 Gew.-%, oder besteht daraus, jeweils bezogen auf das Gesamtgewicht der Emulsion.

### TRÖPFCHENGRÖßE

Gemäß einer weiteren bevorzugten Ausführungsform weisen die in der Wasserphase verteilt vorliegenden Öltröpfchen einen durchschnittlichen volumenbezogenen Durchmesser d_{v 0,1} von 1,0 bis 10,0 µm, bevorzugt 2,0 bis 8,0 µm, weiter bevorzugt 3,0 bis 5,5 µm auf, ebenfalls vorzugsweise bestimmt mittels Laserbeugungsspektroskopie. Besonders bevorzugt weisen die in der Wasserphase verteilt vorliegenden Öltröpfchen einen durchschnittlichen volumenbezogenen Durchmesser d_{v 0,1} von 3,1 bis 5,4 µm auf.

Gemäß einer weiteren bevorzugten Ausführungsform weisen die in der Wasserphase verteilt vorliegenden Öltröpfchen einen durchschnittlichen volumenbezogenen Durchmesser d_{v 0,9} von 1,0 bis 30,0 µm, bevorzugt 5,0 bis 25,0 µm, weiter bevorzugt 10,0 bis 22,0 µm, am bevorzugtesten 10,0 bis 16,0 µm auf, ebenfalls vorzugsweise bestimmt mittels Laserbeugungsspektroskopie. Besonders bevorzugt weisen die in der Wasserphase verteilt vorliegenden Öltröpfchen einen durchschnittlichen volumenbezogenen Durchmesser d_{v 0,9}von 10,1 bis 15,9 µm auf.

Es hat sich nämlich gezeigt, dass die Größe der in der Wasserphase verteilt vorliegenden Öltröpfchen ein Maß für die physikalische Stabilität der Emulsion ist. Eine kleine Größe bedeutet eine geringe Oberflächen- bzw. Grenzflächenspannung und somit eine hohe (physikalische) Stabilität der Emulsion.

Bevorzugt ist die erfindungsgemäße Emulsion frei von Silicadimethylsilylat.

Die Erfindungsgemäße Emulsion umfasst ferner einen oder mehrere von 1,2-Decandiol verschiedene/n Emulgator/en

Es wurde nämlich überraschend herausgefunden, dass 1,2-Decandiol einen coemulgierenden Effekt ausübt und dass durch die Gegenwart von nur geringen Mengen an 1,2-Decandiol in einer Emulsion, die Gesamtemulgatormenge deutlich reduziert werden kann, ohne dabei zugleich die Emulsionsstabilität zu verringern. Besonders überraschend hat sich gezeigt, dass durch die Gegenwart von nur geringen Mengen an 1,2-Decandiol in einer Emulsion, die Gesamtemulgatormenge reduziert werden kann und zugleich die Emulsionsstabilität erhöht werden kann.

### EMULGATOR

Im Rahmen der vorliegenden Erfindung werden Emulgatoren bevorzugt, die einen HLB-Wert im Bereich von 8 bis 18, bevorzugter 8 bis 16, aufweisen.

Der eine oder die mehreren von 1,2-Decandiol verschiedene/n Emulgator/en ist/sind ausgewählt aus der Gruppe bestehend aus PEG-100 Stearat, Cetearyl Glucosid, Distearyldimoniumchlorid, Palmitamidopropyltrimoniumchlorid, Glycerylstearatcitrat, Glyceryloleatcitrat, Polyglyceryl-(3)-Methylglucosedistearat, Cetearylalkohol, Kaliumcetylphosphat, Natriumcetylphosphat, Acrylat/C₁₀-C₃₀-Alkylacrylat-Kreuzpolymer, Ammoniumacryloyldimethyltaurat/Beheneth-25 Methacrylat-Kreuzpolymer, Polyglyceryl-4-Caprat, Polyglyceryl-4-Caprylat/Caprat, Cetyl PEG/PPG-10/1 Dimethicon, Polyglyceryl-6 Dioleat, Polyglyceryl-2-Stearat, PEG-30 Dipoly-hydroxystearat, Natriumstearoyllactylat, PEG-40 hydriertem Rizinusöl, hydrierten Palmglyceriden (INCI-Nomenklatur: hydrogenated Palm Glycerides) oder einer Mischung davon.

Besonders bevorzugt ist/sind der eine oder die mehreren von 1,2-Decandiol verschiedene/n Emulgator/en ausgewählt aus der Gruppe bestehend aus Cetearylalkohol, Glycerylstearatcitrat, Polyglyceryl-(3)-Methylglucosedistearat, Kaliumcetylphosphat, hydrierten Palmglyceriden und einer Mischung davon.

Weiter bevorzugt ist/sind der eine oder die mehreren von 1,2-Decandiol verschiedene/n Emulgator/en ausgewählt aus der Gruppe bestehend aus Glycerylstearatcitrat, Polyglyceryl-(3)-Methylglucosedistearat, Kaliumcetylphosphat, hydrierten Palmglyceriden und einer Mischung davon.

Weiterhin bevorzugt ist der von 1,2-Decandiol verschiedene Emulgator ein Gemisch aus Cetearylalkohol und einem Mitglied der Gruppe bestehend aus (i) einem Gemisch aus Kaliumcetylphosphat und hydrierten Palmglyceriden (bevorzugt im Gewichtsverhältnis im Bereich von 1:1 bis 7:3, insbesondere etwa 6:4 (Kaliumcetylphosphat zu hydrierten Palmglyceriden)), (ii) Glycerylstearatcitrat und (iii) Polyglyceryl-(3)-Methylglucosedistearat.

Weiterhin bevorzugt ist der von 1,2-Decandiol verschiedene Emulgator ein Gemisch aus Cetearylalkohol und einem Gemisch aus Kaliumcetylphosphat und hydrierten Palmglyceriden. Bevorzugt beträgt das Gewichtsverhältnis von Kaliumcetylphosphat zu hydrierten Palmglyceriden in dem Gemisch aus Kaliumcetylphosphat und hydrierten Palmglyceriden 1:1 bis 7:3, insbesondere etwa 6:4.

Gemäß einer weiteren bevorzugten Ausführungsform ist der von 1,2-Decandiol verschiedene Emulgator ein Gemisch aus Cetearylalkohol und Glycerylstearatcitrat. Gemäß einer weiteren bevorzugten Ausführungsform ist der von 1,2-Decandiol verschiedene Emulgator ein Gemisch aus Cetearylalkohol und Polyglyceryl-(3)-Methylglucosedistearat.

Weiterhin bevorzugt ist der von 1,2-Decandiol verschiedene Emulgator ein Gemisch aus Kaliumcetylphosphat und hydrierten Palmglyceriden, wobei das Gewichtsverhältnis von Kaliumcetylphosphat zu hydrierten Palmglyceriden in dem Gemisch aus Kaliumcetylphosphat und hydrierten Palmglyceriden besonders bevorzugt 1:1 bis 7:3, insbesondere etwa 6:4, beträgt.

Weiterhin bevorzugt ist der von 1,2-Decandiol verschiedene Emulgator Glycerylstearatcitrat.

Weiterhin bevorzugt ist der von 1,2-Decandiol verschiedene Emulgator Polyglyceryl-(3)-Methylglucosedistearat.

Der eine oder die mehreren von 1,2-Decandiol verschiedene/n Emulgator/en ist/sind in einer Gesamtmenge von 0,1 bis 5,0 Gew.-%, bevorzugt 0,1 bis 3,0 Gew.-%, weiter bevorzugt 0,5 bis 2,5 Gew.-%, noch weiter bevorzugt 1,0 bis 2,3 Gew.-%, noch weiter bevorzugt 1,0 bis 1,8 am bevorzugtesten 1,6 bis 1,8 Gew.-%, in der Emulsion vorhanden, bezogen auf das Gesamtgewicht der Emulsion.

Gemäß einer bevorzugten Ausführungsform umfasst die erfindungsgemäße Emulsion Cetearylalkohol in einer Menge von 0,1 bis 1,5 Gew.-%, bevorzugt 0,3 bis 1,3 Gew.-%, bevorzugter 0,5 bis 0,9 Gew.-%, besonders bevorzugt etwa 0,7 Gew.-%, bezogen auf das Gesamtgewicht der Emulsion und ferner ein Mitglied der Gruppe bestehend aus (i) einem Gemisch aus Kaliumcetylphosphat und hydrierten Palmglyceriden (bevorzugt im Gewichtsverhältnis im Bereich von 1:1 bis 7:3, insbesondere etwa 6:4 (Kaliumcetylphosphat zu hydrierten Palmglyceriden)), (ii) Glycerylstearatcitrat und (iii) Polyglyceryl-(3)-Methylglucosedistearat in einer Menge von 0,1 bis 5,0 Gew.-%, bevorzugt 0,1 bis 3,0 Gew.-%, weiter bevorzugt 0,5 bis 2,0 Gew.-%, noch weiter bevorzugt 0,5 bis 1,5 Gew.-%, am bevorzugtesten 0,8 bis 1,2 Gew.-%, insbesondere etwa 1,0 Gew.-%, bezogen auf das Gesamtgewicht der Emulsion.

Besonders bevorzugt umfasst die erfindungsgemäße Emulsion Cetearylalkohol in einer Menge von 0,5 bis 0,9 Gew.-% und ferner ein Mitglied der Gruppe bestehend aus (i) einem Gemisch aus Kaliumcetylphosphat und hydrierten Palmglyceriden (bevorzugt im Gewichtsverhältnis im Bereich von 1:1 bis 7:3, insbesondere etwa 6:4 (Kaliumcetylphosphat zu hydrierten Palmglyceriden)), (ii) Glycerylstearatcitrat und (iii) Polyglyceryl-(3)-Methylglucosedistearat in einer Menge von 0,8 bis 1,2 Gew.-%, insbesondere 1,0 Gew.-%, jeweils bezogen auf das Gesamtgewicht der Emulsion.

### 1,2-DECANDIOL

Die erfindungsgemäße Emulsion umfasst 1,2-Decandiol.

Die erfindungsgemäße Emulsion umfasst 1,2-Decandiol in einer Menge im Bereich von 0,005 bis 0,1 Gew.-%. Bevorzugt umfasst die erfindungsgemäße Emulsion 1,2-Decandiol in einer Menge im Bereich von 0,005 bis 0,05 Gew.-% oder von 0,05 bis 0,1 Gew.-%, bezogen auf das Gesamtgewicht der Emulsion.

Bevorzugt umfasst die erfindungsgemäße Emulsion 1,2-Decandiol in einer Menge von 0,03 bis 0,07 Gew.-%, jeweils bezogen auf das Gesamtgewicht der Emulsion.

Es hat sich nämlich gezeigt, dass die Gesamtemulgatormenge einer Emulsion, die 1,2-Decandiol in dieser Menge enthält, signifikant verringert werden kann, ohne gleichzeitig die (physikalische) Stabilität der Emulsion zu reduzieren. Insbesondere hat sich gezeigt, dass durch die Gegenwart von 1,2-Decandiol in dieser geringen Menge die Gesamtemulgatormenge signifikant reduziert werden und gleichzeitig sogar die (physikalische) Stabilität (trotz der reduzierten Gesamtemulgatormenge) erhöht werden kann. Dies ist völlig unerwartet, da eine reduzierte Gesamtemulgatormenge in der Regel zu einer verringerten Stabilität einer Emulsion führt.

Gemäß einer weiteren bevorzugten Ausführungsform umfasst die erfindungsgemäße Emulsion 1,2-Decandiol in einer Menge von 0,005 bis 0,05 Gew.-%, weiter bevorzugt 0,007 bis 0,012 Gew.-%, am bevorzugtesten etwa 0,01 Gew.-%, bezogen auf das Gesamtgewicht der Emulsion. Es hat sich nämlich ferner überraschend gezeigt, dass 1,2-Decandiol bereits in dieser sehr geringen Menge zu einer überaus starken Reduzierung der Oberflächen- bzw. Grenzflächenspannung führt, während andere üblicherweise verwendete 1,2-Alkandiole, wie zum Beispiel 1,2-Pentandiol, 1,2-Hexandiol und 1,2-Octandiol, in dieser Menge zu überhaupt keiner oder nur zu einer vernachlässigbaren Reduzierung der Oberflächen- bzw. Grenzflächenspannung führen.

Es ist bevorzugt, dass die erfindungsgemäße Emulsion einen pH-Wert von 4,0 bis 8,0, bevorzugt 5,0 bis 6,5 aufweist.

### WEITERE BESTANDTEILE

Die erfindungsgemäße Emulsion kann ferner weitere nützliche Bestandteile enthalten, wie zum Beispiel:
Konservierungsmittel, antimikrobielle Mittel wie z.B weitere antibakterielle Mittel oder Fungizide, Abrasiva, Antiakne-Mittel, Mittel gegen Hautalterung, Anticellulitis-Mittel, Antischuppen-Mittel, entzündungshemmende Mittel, irritationsverhindernde Mittel, irritationshemmende Mittel, Antioxidantien, Adstringentien, schweisshemmende Mittel, antiseptische Mittel, Antistatika, Binder, Puffer, Trägermaterialien, Chelatbildner, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Slilcone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UV-absorbierende Mittel, UV-Filter, Waschmittel, Weichspüler, suspendierende Mittel, Hautbräunungsmittel, Verdickungsmittel, rheologische Additive, Vitamine, Öle, Wachse, Fette, Phospholipide, gesättigte Fettsäuren, ein- oder mehrfach ungesättigte Fettsäuren, α-Hydroxysäuren, Polyhydroxyfettsäuren, Verflüssiger, Farbstoffe, farbschützende Mittel, Pigmente, Antikorrosiva, Aromen, Geschmackstoffe, Riechstoffe, Polyole, Tenside, Elektrolyte, organische Lösungsmittel oder Silikonderivate.

Die erfindungsgemäße kosmetische Emulsion kann auch schweißhemmende Wirkstoffe (Antitranspirantien) enthalten und zum Beispiel als Deodorant zur Bekämpfung von Körpergeruch eingesetzt werden. Als schweißhemmende Wirkstoffe kommen vor allem Aluminiumsalze wie Aluminiumchlorid, Aluminiumchlorhydrat, -nitrat, -sulfat, -acetat usw. zum Einsatz. Daneben kann aber auch die Verwendung von Zink-, Magnesium- und Zirkoniumverbindungen vorteilhaft sein. Insbesondere haben sich im Wesentlichen die Aluminiumsalze und - in etwas geringerem Maße - Aluminium/Zirkoniumsalz-Kombinationen bewährt. Daneben erwähnenswert sind die teilneutralisierten und damit besser hautverträglichen, aber nicht ganz so wirksamen Aluminiumhydroxychloride.

Bevorzugt wirkt die erfindungsgemäße Emulsion zusätzlich als Sonnenschutzmittel. Dazu enthält die erfindungsgemäße Emulsion bevorzugt mindestens einen UVA-Filter und/oder mindestens einen UVB-Filter und/oder mindestens einen Breitbandfilter und/oder mindestens ein anorganisches Pigment.

Bevorzugt enthält die erfindungsgemäße Emulsion mindestens einen UVA-Filter und/oder mindestens einen UVB-Filter und/oder mindestens einen Breitbandfilter und/oder mindestens ein anorganisches Pigment in einer Gesamtmenge von 0,01 Gew.-% bis 40 Gew.-%, vorzugsweise 0,1% bis 10 Gew.-%, insbesondere 1,0 bis 5,0 Gew.-% beträgt, bezogen auf das Gesamtgewicht der Emulsion.

### VITAMINE

Bevorzugt enthält die erfindungsgemäße Emulsion Vitamine und/oder Vitaminvorstufen, wobei alle für kosmetische und/oder dermatologische Anwendungen geeigneten oder gebräuchlichen Vitamine und Vitaminvorstufen verwendet werden können. Erwähnenswert sind hier insbesondere Vitamine und Vitaminvorstufen wie Tocopherole, Vitamin A, Niacinsäure und Niacinsäureamid, weitere Vitamine des B-Komplexes, insbesondere Biotin und Vitamin C, Panthenol und dessen Derivate, insbesondere die Ester und Ether des Panthenols sowie kationisch derivatisierte Panthenole wie z.B. Panthenoltriacetat, Panthenolmonoethylether und dessen Monoacetat sowie kationische Panthenolderivate.

### RHEOLOGISCHE ADDITIVE

Besonders bevorzugt enthält die erfindungsgemäße kosmetische Emulsion ferner ein oder mehrere rheologische/s Additiv/e. Unter rheologischen Additiven versteht man Zusatzstoffe, welche in der kosmetischen Emulsion einen rheologischen Effekt, wie z.B. eine Erhöhung der Viskosität, ausüben.

Geeignete rheologische Additive sind beispielsweise polymere Verbindungen wie zum Beispiel solche des Aerosil^{®}-Typs (hydrophiles Siliziumdioxid), Polysaccharide, bevorzugt Xanthangummi, Guar-Gummi, Agar-Agar, Alginate und Tylosen, Carboxymethylcellulose und Hydroxyethylcellulose, Polyethylenglycol-Monoester und -Diester von Fettsäuren mit relativ hohem Molekulargewicht, Polyacrylate (zum Beispiel Carbomer, Carbopole^{®} [Goodrich] or Synthalene^{®} [Sigma]), Polyacrylamide, Polyvinylalkohol und Polyvinylpyrrolidon, Tenside wie zum Beispiel ethoxylierte Fettsäureglyceride, Ester von Fettsäuren mit Polyolen, zum Beispiel Pentaerythritol oder Trimethylolpropan, schmalbandige Fettalkoholethoxylate und Elektrolyte wie zum Beispiel Natriumchlorid und Ammoniumchlorid, oder eine Mischung davon.

Bevorzugt werden Carbomer und/oder Xanthangummi als rheologische Additive verwendet. Sie zählen zu den hydrophilen Gelbildern und bilden ein zusammenhängendes dreidimensionales Netzwerk, welches Flüssigkeit bindet. Carbomer und Xanthangummi verdicken die kontinuierliche Phase der erfindungsgemäßen Emulsion, was die (physikalische) Stabilität der Emulsion weiter erhöht.

Wenn Carbomer (dies ist ein Acrylsäurepolymerisat) als rheologisches Additiv verwendet wird, weist die erfindungsgemäße Emulsion bevorzugt einen pH-Wert im Bereich von 5 bis 11, weiter bevorzugt 5 bis 10, besonders bevorzugt 5,5 bis 6,5, auf. Denn die aus dem Carbomer resultierende Viskosität ist zusätzlich zur Einsatzmenge von dem eingestellten pH-Wert abhängig, wobei die maximale Viskosität bei einem pH-Wert von 6 bis 10 erreicht wird. Wenn Carbomer als rheologisches Additiv eingesetzt wird, ist die erfindungsgemäße Emulsion bevorzugt frei von kationischen Stoffen und/oder Elektrolyten und/oder mehrwertigen Metallionen.

Zur Einstellung des pH-Wertes wird bevorzugt eine wässrige Natriumhydroxidlösung, bevorzugt 10%ig (Massenprozent), verwendet.

Das/die rheologische/n Additiv/e sind bevorzugt jeweils in einer Menge von 0,01 bis 3,0 Gew.-%, bevorzugt 0,01 bis 2,0 Gew.-%, bevorzugter 0,01 bis 1,0 Gew.-%, weiter bevorzugt 0,01 bis 0,5 Gew.-%, weiter bevorzugt 0,05 bis 0,15 Gew.-%, am bevorzugtesten etwa 0,1 Gew.-%, in der erfindungsgemäßen Emulsion vorhanden, bezogen auf das Gesamtgewicht der Emulsion.

Besonders bevorzugt umfasst die erfindungsgemäße Emulsion Carbomer und/oder Xanthangummi in einer Menge von jeweils 0,1 Gew.-%, bezogen auf das Gesamtgewicht der Emulsion.

Die erfindungsgemäße Emulsion ist bevorzugt ein Hautpflegemittel, Haarpflegemittel oder ein Sonnenschutzmittel. Bevorzugt liegt die erfindungsgemäße in Form einer Creme, einer Lotion, eines Sprays, einer Salbe oder eines Gels vor. Besonders bevorzugt ist die erfindungsgemäße Emulsion eine Hautcreme, ein Haarwaschmittel, ein Duschgel, ein Antitranspirant, ein Deodorant oder eine Seife.

### VERWENDUNG

Die vorliegende Erfindung beschreibt ferner die Verwendung von 1,2-Decandiol zum Herabsetzen der Oberflächen- bzw. Grenzflächenspannung einer kosmetischen Emulsion. Bevorzugt wird 1,2-Decandiol in einer oben beschriebenen erfindungsgemäßen Emulsion zum Herabsetzen der Oberflächen- bzw. Grenzflächenspannung verwendet.

Es wurde nämlich überraschend herausgefunden, dass 1,2-Decandiol die Oberflächen- bzw Grenzflächenspannung außerordentlich stark herabsetzt. Dies ermöglicht einen Einsatz von 1,2-Decandiol in sehr geringen Mengen, insbesondere in Mengen, in denen andere 1,2-Alkandiole, wie zum Beispiel 1,2-Pentandiol, 1,2-Hexandiol und 1,2-Octandiol, die Oberflächen- bzw. Grenzflächenspannung gar nicht oder nur in einem sehr geringen Ausmaß herabsetzen.

Die vorliegende Erfindung betrifft ferner die Verwendung von 1,2-Decandiol als Co-Emulgator zum Verringern der Gesamtemulgatormenge in einer kosmetischen Emulsion. Bevorzugt wird 1,2-Decandiol in einer oben beschriebenen erfindungsgemäßen Emulsion als Co-Emulgator zum Verringern der Gesamtemulgatormenge verwendet.

Es wurde nämlich überraschend herausgefunden, dass die Gesamtemulgatormenge einer Emulsion, die eine geringe Menge von 1,2-Decandiol enthält, signifikant verringert werden kann, ohne gleichzeitig die (physikalische) Stabilität zu reduzieren.

### VERFAHREN

Die vorliegende Erfindung betrifft ferner ein Verfahren zur Herstellung der erfindungsgemäßen Emulsion. Das Verfahren umfasst die folgenden Schritte oder besteht daraus: a) Bereitstellen einer wässrigen Phase; b) Bereitstellen einer Ölphase; c) Kombinieren der wässrigen Phase und der Ölphase, wobei die wässrige Phase und/oder die Ölphase 1,2-Decandiol umfasst.

Gemäß einer Ausführungsform umfasst die wässrige Phase 1,2-Decandiol. Gemäß einer weiteren Ausführungsform umfasst die Ölphase 1,2-Decandiol. Gemäß einer weiteren Ausführungsform umfassen die wässrige Phase und die Ölphase 1,2-Decandiol.

Die wässrige Phase und/oder die Ölphase wird/ werden vor Schritt c) erwärmt, was zu einer guten Homogenisierung der Phasen führt.

In diesem Zusammenhang wurde herausgefunden, dass eine besonders gute Homogenisierung erreicht wird, wenn die Phasen auf eine Temperatur von 50 bis 100°C erwärmt werden, insbesondere, wenn die wässrige Phase auf eine Temperatur im Bereich von 70 bis 90°C, vorzugsweise auf etwa 80°C, erwärmt wird und wenn die Ölphase auf eine Temperatur im Bereich von 80 bis 100°C, vorzugsweise auf etwa 90°C, erwärmt wird.

Daher werden die Phasen bevorzugt auf eine Temperatur von 50 bis 100°C erwärmt. Besonders bevorzugt wird die wässrige Phase auf eine Temperatur im Bereich von 70 bis 90°C, vorzugsweise auf etwa 80°C, erwärmt und die Ölphase wird auf eine Temperatur im Bereich von 80 bis 100°C, vorzugsweise auf etwa 90°C, erwärmt. Dies führte zu den besten Ergebnissen.

Wenn die erfindungsgemäße Emulsion ein oder mehrere rheologische/s Additiv/e umfasst, wird bevorzugt zunächst die Ölphase durch Kombinieren und Mischen der einzelnen Bestandteile, aber ohne das/die rheologische/n Additiv/e, bereitgestellt.

Danach wird dieses Gemisch erwärmt, bevorzugt auf die oben angegebene Temperatur und erst danach wird/werden das/die rheologische/n Additiv/e hinzugegeben.

Die in Schritt c) kombinierten Phasen werden homogenisiert. Dazu wird eine Zahnkranzdispergier-Einheit (IKA^{®} T25 digital ULTRA TURRAX^{®}) verwendet. Besonders bevorzugt erfolgt die Homogenisierung für drei Minuten bei 6000 U/min.

Anschließend (also nach erfolgter Homogenisierung) kann die erhaltene Emulsion unter Rühren, bevorzugt mittels eines Blattrührers, besonders bevorzugt bei 150 U/min, mit einer Natriumhydroxidlösung, bevorzugt 10%ig (Massenprozent), auf den gewünschten pH-Wert eingestellt werden. Wenn Carbomer als rheologisches Additiv verwendet wird, wird die erhaltene Emulsion bevorzugt auf einen pH-Wert im Bereich von 5-11, bevorzugt 5-10, besonders bevorzugt 5,5 bis 6,5, eingestellt.

Dieser pH-Wert hat zudem den Vorteil, dass der Säureschutzmantel der Haut nicht angegriffen wird, was zu einer guten Hautverträglichkeit führt.

Nach Einstellen des gewünschten pH-Werts wird die Emulsion, bevorzugt schonend, unter weiterem Rühren für 10 Minuten bei 150 U/min, 10 Minuten bei 100 U/min und 5 Minuten bei 50 U/min abgekühlt, bis sie nach Abschluss des Rührvorgangs eine Temperatur von etwa 43°C aufweist.

### BEISPIELE

Nachfolgend werden bevorzugte Ausgestaltungen der vorliegenden Erfindung anhand von Beispielen näher erläutert.

### Beispiel 1

### Einfluss von verschiedenen 1,2-Alkandiolen auf die Oberflächenspannung

Als 1,2-Alkandiole wurden 1,2-Pentandiol, 1,2-Hexandiol, 1,2-Octandiol und 1,2-Decandiol verwendet. Als Medium wurde deionisiertes Wasser verwendet.

Die Oberflächenspannung von wässrigen Lösungen der verschiedenen 1,2-Alkandiole wurde mit einem Tropfenvolumentensiometer (LAUDA TVT2) nach dem Prinzip der Tropfen-Volumen-Methode bestimmt. Die Präzisionsspritze, die Kapillare und das Wasser wurden auf eine Temperatur von 20 ± 1°C temperiert.

Der Volumenstrom betrug 0,5 Sekunden pro Mikroliter [s/µl]. Der Radius der verwendeten Kapillare betrug 0,39 mm.

Die 1,2-Alkandiole wurden in folgenden Mengen eingesetzt (Gew.-%, bezogen auf das Gesamtgewicht des Gemisches):

**Tabelle 1**

| **Lösung** | **1,2-Alkandiol** | **Menge [Gew.-%]** |
|---|---|---|
| 1 | 1,2-Pentandiol | 0,01; 0,5 |
| 2 | 1,2-Hexandiol | 0,01; 0,5 |
| 3 | 1,2-Octandiol | 0,01; 0,5 |
| 4 | 1,2-Decandiol | 0,01 |

Die geringe Löslichkeit von 1,2-Decandiol in Wasser erlaubte keine Messung bei einer Menge von 0,5 Gew.-%. Die Messungen der Lösungen 1 bis 3 bei einer Menge von 0,5 Gew.-% dienten dazu, zu verifizieren dass diese 1,2-Alkandiole (1,2-Pentandiol, 1,2-Hexandiol und 1,2-Octandiol) die Oberflächenspannung grundsätzlich herabsetzen können, dazu aber höhere Mengen als 0,01 Gew.-% benötigt werden (siehe unten).

Die Oberflächenspannungsmessung wird in Dreifachbestimmung durchgeführt, wobei jede Einfachmessung der Mittelwert aus den Messwerten fünf abfallender Tropfen ist. Die in Tabelle 2 gezeigten Werte berechnen sich durch eine Mittelwertbildung der Dreifachbestimmungen.

Die Ergebnisse sind in Tabelle 2 gezeigt:

**Tabelle 2**

| | **Oberflächenspannung [mN/m]** | | |
|---|---|---|---|
| | **0 Gew.-% *** | **0,01 Gew.-%** | **0,5 Gew.-%** |
| 1,2-Pentandiol | 69,23 | 69,51 | 64,80 |
| 1,2-Hexandiol | 69,23 | 69,31 | 55,93 |
| 1,2-Octandiol | 69,23 | 66,64 | 27,97 |
| 1,2-Decandiol | 69,23 | 38,25 | - |

| | | | |
|---|---|---|---|
| * Vergleichsbeispiel | | | |

Es hat sich gezeigt, dass 1,2-Decandiol bereits bei einer Menge von nur 0,01 Gew.-% die Oberflächenspannung von Wasser (dieses prägt den Grundcharakter einer O/W-Emulsion) außerordentlich stark herabsetzt, wohingegen 1,2-Pentandiol und 1,2-Hexandiol zu keiner Herabsetzung der Oberflächenspannung führen und 1,2-Octandiol zu einer vernachlässigbaren Herabsetzung der Oberflächenspannung führt.

Die Tatsache, dass 1,2-Pentandiol und 1,2-Hexandiol bei einer Menge von 0,01 Gew.-% eine leichte Zunahme der Oberflächenspannung zeigen, ist wahrscheinlich auf Messungenauigkeiten zurückzuführen. Dass auch diese 1,2-Alkandiole die Oberflächenspannung tatsächlich herabsetzen, wurde durch Messungen bei einer Menge von 0,5 Gew.-% verifiziert. Dabei ist erstaunlicherweise zu sehen, dass 1,2-Pentandiol und 1,2-Hexandiol bei einer Menge von 0,5 Gew.-% die Oberflächenspannung nicht annähernd so stark herabsetzen wie 1,2-Decandiol bei einer Menge von nur 0,01 Gew.-%. Selbst 1,2-Octandiol, das sich lediglich durch zwei zusätzliche CH₂-Gruppen von 1,2-Decandiol unterscheidet, setzt die Oberflächenspannung bei einer Menge von 0,5 Gew.-% nur unwesentlich stärker herab wie 1,2-Decandiol bei einer Menge von nur 0,01 Gew.-%.

### Beispiel 2

### Co-emulgierender Effekt von 1,2-Decandiol und Reduktion der Gesamtemulgatormenge.

Der Co-emulgierende Effekt von 1,2-Decandiol und die Möglichkeit, die Gesamtemulgatormenge einer Emulsion durch die Zugabe von nur geringen Mengen an 1,2-Decandiol signifikant herabzusetzen ohne dabei gleichzeitig die (physikalische) Stabilität zu verringern wurde anhand von drei marktrelevanten Emulgatoren/Emulgatorsystemen untersucht, nämlich
(i) Cetearylalkohol und ein Gemisch aus Kaliumcetylphosphat und hydrierten Palmglyceriden (das Gewichtsverhältnis von Kaliumcetylphosphat zu hydrierten Palmglyceriden im Gemisch aus Kaliumcetylphosphat und hydrierten Palmglyceriden betrug 6:4),
(ii) Cetearylalkohol und Glycerylstearatcitrat und
(iii) Cetearylalkohol und Polyglyceryl-(3)-Methylglucosedistearat.

Es wurden die in den Tabellen 3 bis 5 gezeigten Modellemulsionen hergestellt. Die Quelle der einzelnen Bestandteile sind in den folgenden Tabellen angegeben.

**Tabelle 3**

| **Phase** | **Bestandteil** | **Emulsion** | | | |
|---|---|---|---|---|---|
| | | **1*** | **2** | **3** | **4** |
| **A** | Kaliumcetylphosphat, hydrierte Palmglyceride (INCI-Nomenklatur: Potassium Cetyl Phosphate, Hydrogenated Palm Glycerides) | 2,0 Gew.-% | 2,0 Gew.-% | 1,0 Gew.-% | 1,0 Gew.-% |
| | 1,2-Decandiol, ex Symrise AG | - | 0,1 Gew.-% | 0,1 Gew.-% | 0,05 Gew.-% |
| | Cetearylalkohol, ex BASF | 0,7 Gew.-% | 0,7 Gew.-% | 0,7 Gew.-% | 0,7 Gew.-% |
| | Caprylic/Capric Triglycerid, ex IOI | 8,0 Gew.-% | 8,0 Gew.-% | 8,0 Gew.-% | 8,0 Gew.-% |
| | Cetearylethylhexanoat, ex Symrise AG | 4,0 Gew.-% | 4,0 Gew.-% | 4,0 Gew.-% | 4,0 Gew.-% |
| | Dimethicon, ex Evonik | 0,1 Gew.-% | 0,1 Gew.-% | 0,1 Gew.-% | 0,1 Gew.-% |
| **B** | Wasser (deionisiert) | 83,4 Gew.-% | 83,3 Gew.-% | 84,3 Gew.-% | 84,35 Gew.-% |
| | Glycerin, ex IOI | 1,8 Gew.-% | 1,8 Gew.-% | 1,8 Gew.-% | 1,8 Gew.-% |
| | pH-Wert | 5,9 | 5,8 | 5,9 | 5,9 |

| | | | | | |
|---|---|---|---|---|---|
| * Vergleichsbeispiel | | | | | |

**Tabelle 4**

| **Phase** | **Bestandteil** | **Emulsion** | | | |
|---|---|---|---|---|---|
| | | **5*** | **6** | **7** | **8** |
| **A** | Glycerylstearatcitrat, ex Symrise AG | 2,0 Gew.-% | 2,0 Gew.-% | 1,0 Gew.-% | 1,0 Gew.-% |
| | 1,2-Decandiol, ex Symrise AG | - | 0,1 Gew.-% | 0,1 Gew.-% | 0,05 Gew.-% |
| | Cetearylalkohol, ex BASF | 0,7 Gew.-% | 0,7 Gew.-% | 0,7 Gew.-% | 0,7 Gew.-% |
| | Caprylic/Capric Triglycerid, ex IOI | 8,0 Gew.-% | 8,0 Gew.-% | 8,0 Gew.-% | 8,0 Gew.-% |
| | Cetearylethylhexanoat, ex Symrise AG | 4,0 Gew.-% | 4,0 Gew.-% | 4,0 Gew.-% | 4,0 Gew.-% |
| | Dimethicon, ex Evonik | 0,1 Gew.-% | 0,1 Gew.-% | 0,1 Gew.-% | 0,1 Gew.-% |
| B | Wasser (deionisiert) | 83,4 Gew.-% | 83,3 Gew.-% | 84,3 Gew.-% | 84,35 Gew.-% |
| | Glycerin, ex IOI | 1,8 Gew.-% | 1,8 Gew.-% | 1,8 Gew.-% | 1,8 Gew.-% |
| | pH-Wert | 6,2 | 6,1 | 6,2 | 6,1 |

| | | | | | |
|---|---|---|---|---|---|
| * Vergleichsbeispiel | | | | | |

**Tabelle 5**

| **Phase** | **Bestandteil** | **Emulsion** | | |
|---|---|---|---|---|
| | | **9*** | **10** | **11** |
| **A** | Polyglyceryl-(3)-Methylglucosedistearat, ex Evonik | 2,0 Gew.-% | 2,0 Gew.-% | 1,0 Gew.-% |
| | 1,2-Decandiol, ex Symrise AG | - | 0,1 Gew.-% | 0,1 Gew.-% |
| | Cetearylalkohol, ex BASF | 0,7 Gew.-% | 0,7 Gew.-% | 0,7 Gew.-% |
| | Caprylic/Capric Triglycerid, ex IOI | 8,0 Gew.-% | 8,0 Gew.-% | 8,0 Gew.-% |
| | Cetearylethylhexanoat, ex Symrise AG | 4,0 Gew.-% | 4,0 Gew.-% | 4,0 Gew.-% |
| | Dimethicon, ex Evonik | 0,1 Gew.-% | 0,1 Gew.-% | 0,1 Gew.-% |
| **B** | Wasser (deionisiert) | 83,4 Gew.-% | 83,3 Gew.-% | 84,3 Gew.-% |
| | Glycerin, ex IOI | 1,8 Gew.-% | 1,8 Gew.-% | 1,8 Gew.-% |
| | pH-Wert | 7,1 | 7,0 | 6,9 |

| | | | | |
|---|---|---|---|---|
| * Vergleichsbeispiel | | | | |

Die Modellemulsionen wurden folgendermaßen hergestellt: Die Bestandteile der Phasen A (Ölphase) und B (Wasserphase) wurden vermischt und Phasen A und B wurden jeweils auf 80°C erwärmt. Anschließend wurde Phase B zu Phase A gegeben und mittels eines IKA T25 digital Ultra TURRAX Mixers für 3 Minuten bei 6000 U/min emulgiert. Die Emulsionen wurden unter Rühren mit einem Flügelrührer auf Raumtemperatur gekühlt.

Da die Größe der in der kontinuierlichen Wasserphase verteilt vorliegenden Öltröpfchen ein Maß für die (physikalische) Stabilität der Emulsion ist, wurde die Öltröpfchengröße (volumenbezogener Durchmesser) der verschiedenen Modellemulsionen bestimmt.

Die Öltröpfchengröße (volumenbezogener Durchmesser) wurde mit einem Gerät des Typs Malvern "Mastersizer 3000" bestimmt. Lösemittel: Wasser/Ethanol (90:10 (Volumenverhältnis), Sättigung: maximal 10%, Pumpengeschwindigkeit: 1940 U/min, Dispersionsdauer: 2 Minuten.

Die Ergebnisse sind in Tabelle 6 sowie in den Figuren 1 bis 11 veranschaulicht. Figuren 1 bis 11 zeigen Lichtmikroskopaufnahmen der Emulsionen 1 bis 11 (aufgenommen mit einem Lichtmokroskop des Typs Olympus IX-70, 60-fache Vergrößerung).
- Figur 1: zeigt eine Lichtmikroskopaufnahme der Emulsion 1.
- Figur 2: zeigt eine Lichtmikroskopaufnahme der Emulsion 2.
- Figur 3: zeigt eine Lichtmikroskopaufnahme der Emulsion 3.
- Figur 4: zeigt eine Lichtmikroskopaufnahme der Emulsion 4.
- Figur 5: zeigt eine Lichtmikroskopaufnahme der Emulsion 5.
- Figur 6: zeigt eine Lichtmikroskopaufnahme der Emulsion 6.
- Figur 7: zeigt eine Lichtmikroskopaufnahme der Emulsion 7.
- Figur 8: zeigt eine Lichtmikroskopaufnahme der Emulsion 8.
- Figur 9: zeigt eine Lichtmikroskopaufnahme der Emulsion 9.
- Figur 10: zeigt eine Lichtmikroskopaufnahme der Emulsion 10.
- Figur 11: zeigt eine Lichtmikroskopaufnahme der Emulsion 11.

**Tabelle 6**

| **Emulsion** | **d _{v 0,1} [µm]** | **d _{v 0,5} [µm]** | **d _{v 0,9} [µm]** |
|---|---|---|---|
| 1* | 5,14 | 9,15 | 15,95 |
| 2 | 3,79 | 7,30 | 13,25 |
| 3 | 4,18 | 7,22 | 12,10 |
| 4 | 4,33 | 7,61 | 13,90 |
| | | | |
| 5* | 5,67 | 10,05 | 16,65 |
| 6 | 5,35 | 9,59 | 15,90 |
| 7 | 4,21 | 7,58 | 12,85 |
| 8 | 4,56 | 8,02 | 13,60 |
| | | | |
| 9* | 4,74 | 9,59 | 18,10 |
| 10 | 3,86 | 8,35 | - |
| 11 | 3,21 | 5,86 | 10,45 |

| | | | |
|---|---|---|---|
| * Vergleichsbeispiel | | | |

Wie aus Tabelle 6 hervorgeht, weisen die erfindungsgemäßen (d.h. 1,2-Decandiol-haltigen) Emulsionen eine verringerte Öltröpfchengröße und damit eine erhöhte (physikalische) Stabilität als die Vergleichsemulsionen (Emulsionen 1, 5, 9) auf.

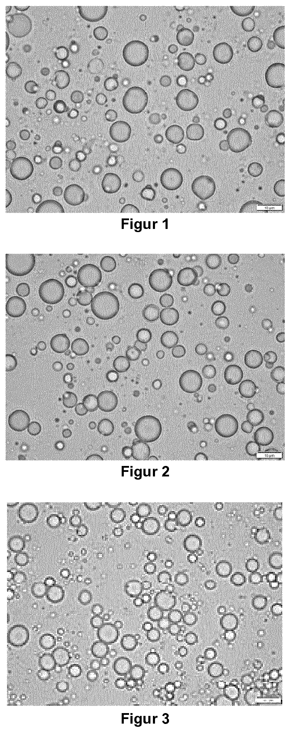

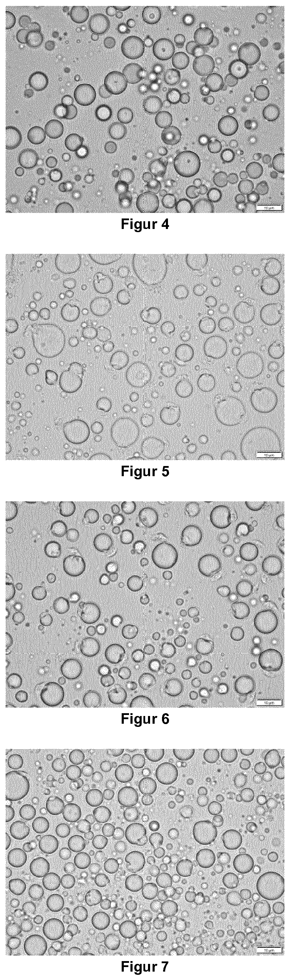

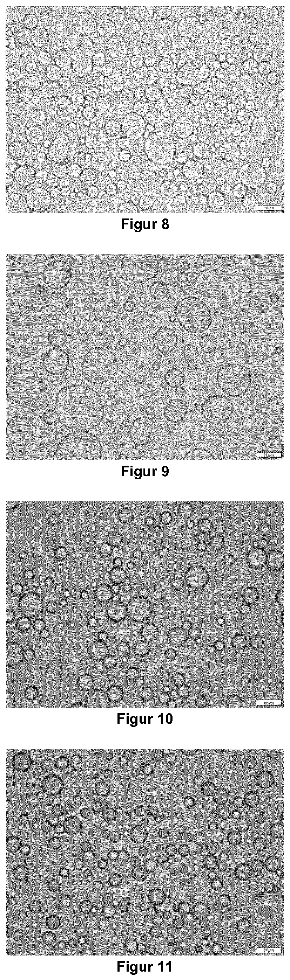

Insbesondere geht aus Tabelle 6 sowie Figuren 1 bis 11 hervor, dass die Gegenwart von nur 0,1 Gew.-% bzw. 0,05 Gew.-% 1,2-Decandiol es ermöglicht, die Gesamtemulgatormenge signifikant (um mehr als 30%) zu verringern, ohne dabei gleichzeitig die (physikalische) Stabilität der Emulsion zu verringern. Dies geht vor allem aus einem Vergleich der Emulsionen 3, 4 und 1*, der Emulsionen 7, 8 und 5* sowie der Emulsionen 11 und 9* hervor. Besonders überraschend wurde herausgefunden, dass die 1,2-Decandiol-haltigen Emulsionen trotz der verringerten Gesamtemulgatormenge sogar eine höhere Stabilität (d.h. geringere Öltröpfchengröße) als die Vergleichsemulsionen aufweisen.

## Patentansprüche

1. Kosmetische Emulsion, umfassend:
eine kontinuierliche Wasserphase,
in der kontinuierlichen Wasserphase verteilt vorliegende Öltröpfchen und
1,2-Decandiol,
wobei die in der kontinuierlichen Wasserphase verteilt vorliegenden Öltröpfchen einen durchschnittlichen volumenbezogenen Durchmesser d_{v 0,5} von 1,0 bis 10,0 µm, bevorzugt 3,0 bis 10,0 µm, weiter bevorzugt 5,0 bis 10,0 µm, aufweisen
und
wobei die Emulsion ferner einen oder mehrere von 1,2-Decandiol verschiedene/n Emulgator/en umfasst
und
wobei der eine oder die mehreren von 1,2-Decandiol verschiedene/n Emulgator/en in einer Gesamtmenge von 0,1 bis 5,0 Gew.-%, bevorzugt 0,1 bis 3,0 Gew.-%, weiter bevorzugt 0,5 bis 2,5 Gew.-%, noch weiter bevorzugt 1,0 bis 2,3 Gew.-%, noch weiter bevorzugt 1,0 bis 1,8 Gew.-%, am bevorzugtesten 1,6 bis 1,8 Gew.-%, in der Emulsion vorhanden sind, bezogen auf das Gesamtgewicht der Emulsion
und
wobei 1,2-Decandiol in einer Menge im Bereich von 0,005 bis 0,1 Gew.-%, bevorzugt von 0,005 bis 0,05 Gew.-% oder von 0,05 bis 0,1 Gew.-%, in der Emulsion vorhanden ist, bezogen auf das Gesamtgewicht der Emulsion,
und
wobei der eine oder die mehreren von 1,2-Decandiol verschiedene/n Emulgator/en ausgewählt ist/sind aus der Gruppe bestehend aus PEG-100 Stearat, Cetearyl Glucosid, Distearyldimoniumchlorid, Palmitamidopropyl-trimoniumchlorid, Glycerylstearatcitrat, Glyceryloleatcitrat, Polyglyceryl-(3)-Methylglucosedistearat, Cetearylalkohol, Kaliumcetylphosphat, Natriumcetylphosphat, Acrylat/C10-C30-Alkylacrylat-Kreuzpolymer, Ammoniumacryloyldimethyltaurat/Beheneth-25 Methacrylat-Kreuzpolymer, Polyglyceryl-4-Caprat, Polyglyceryl-4-Caprylat/Caprat, Cetyl PEG/PPG-10/1 Dimethicon, Polyglyceryl-6 Dioleat, Polyglyceryl-2-Stearat, PEG-30 Dipoly-hydroxystearat, Natriumstearoyllactylat, PEG-40 hydriertes Rizinusöl, hydrierten Palmglyceriden oder einer Mischung davon.

2. Emulsion nach Anspruch 1, wobei die Emulsion frei von Silicadimethylsilylat ist.

3. Emulsion nach Anspruch 1 oder 2, wobei der eine oder die mehreren von 1,2-Decandiol verschiedene/n Emulgator/en ausgewählt ist/sind aus der Gruppe bestehend aus Cetearylalcohol, Glycerylstearatcitrat, Polyglyceryl-(3)-Methylglucosedistearat, Kaliumcetylphosphat, hydrierten Palmglyceriden und einer Mischung davon.

4. Emulsion nach einem der vorangehenden Ansprüche, wobei die Emulsion ein Zweiphasensystem aus einem kosmetischen Öl in Wasser ist.

5. Emulsion nach einem der vorangehenden Ansprüche, wobei die Emulsion weniger als 25 Gew.-%, bevorzugt weniger als 20 Gew.-%, weiter bevorzugt weniger als 10 Gew.-%, besonders bevorzugt weniger als 5 Gew.-%, am bevorzugtesten 0 Gew.-%, silikonhaltige Öle aufweist, bezogen auf das Gesamtgewicht der Emulsion.

6. Kosmetische Emulsion nach einem der vorangehenden Ansprüche, wobei die in der Wasserphase verteilt vorliegenden Öltröpfchen einen durchschnittlichen volumenbezogenen Durchmesser d_{v 0,1} von 1,0 bis 10,0 µm, bevorzugt 2,0 bis 8,0 µm, weiter bevorzugt 3,0 bis 5,5 µm aufweisen, bestimmt mittels Laserbeugungsspektroskopie.

7. Kosmetische Emulsion nach einem der vorangehenden Ansprüche, wobei die in der Wasserphase verteilt vorliegenden Öltröpfchen einen durchschnittlichen volumenbezogenen Durchmesser d_{v 0,9} von 1,0 bis 30,0 µm, bevorzugt 5,0 bis 25,0 µm, weiter bevorzugt 10,0 bis 22,0 µm, am bevorzugtesten 10,0 bis 16,0 µm aufweisen, bestimmt mittels Laserbeugungsspektroskopie.

8. Verwendung von 1,2-Decandiol als Co-Emulgator zum Verringern der Gesamtemulgatormenge in einer Emulsion nach einem der Ansprüche 1 bis 7.

9. Verfahren zur Herstellung einer kosmetischen Emulsion gemäß einem der Ansprüche 1 bis 7, bestehend aus oder umfassend die folgenden Schritte:
a) Bereitstellen einer wässrigen Phase;
b) Bereitstellen einer Ölphase;
c) Kombinieren der wässrigen Phase und der Ölphase,
wobei die wässrige Phase und/oder die Ölphase 1,2-Decandiol umfasst,
und
wobei die wässrige Phase und/oder die Ölphase vor Schritt c) erwärmt wird / werden, bevorzugt auf eine Temperatur von 50 bis 100°C, bevorzugt wobei die wässrige Phase auf eine Temperatur im Bereich von 70 bis 90 °C, vorzugsweise auf etwa 80°C, erwärmt wird und die Ölphase auf eine Temperatur im Bereich von 80 bis 100 °C, vorzugsweise auf etwa 90°C,
und
wobei die kombinierten Phasen nach Schritt c) mittels einer Zahnkranzdispergier-Einheit homogenisiert werden.

## Claims

1. A cosmetic emulsion comprising:
a continuous aqueous phase,
oil droplets dispersed in the continuous aqueous phase, and
1,2-decanediol,
wherein the oil droplets dispersed in the continuous aqueous phase have an average volume-based diameter d_{v0.5} of 1.0 to 10.0 µm, preferably 3.0 to 10.0 µm, more preferably 5.0 to 10.0 µm,
and
wherein the emulsion further comprises one or more emulsifier(s) other than 1,2-decanediol
and
wherein the one or more emulsifier(s) other than 1,2-decanediol is/are present in the emulsion in a total amount of 0.1 to 5.0 wt.-%, preferably 0.1 to 3.0 wt.-%, more preferably 0.5 to 2.5 wt.-%, even more preferably 1.0 to 2.3 wt.-%, most preferably 1.0 to 1.8 wt.-%, even most preferably 1.6 to 1.8 wt.-%, based on the total weight of the emulsion,
and
wherein 1,2-decanediol is present in the emulsion in an amount in the range of 0.005 to 0.1 wt.-%, preferably from 0.005 to 0.05 wt.-% or from 0.05 to 0.1 wt.-%, based on the total weight of the emulsion,
and
wherein the one or more emulsifier(s) other than 1,2-decanediol is/are selected from the group consisting of PEG-100 stearate, cetearyl glucoside, distearyldimonium chloride, palmitamidopropyltrimonium chloride, glyceryl stearate citrate, glyceryl oleate citrate, polyglyceryl-(3)-methylglucose distearate, cetearyl alcohol, potassium cetyl phosphate, sodium cetyl phosphate, acrylates/C10-C30 alkyl acrylate crosspolymer, ammonium acryloyldimethyltaurate/beheneth-25 methacrylate crosspolymer, polyglyceryl-4 caprate, polyglyceryl-4 caprylate/caprate, cetyl PEG/PPG-10/1 dimethicone, polyglyceryl-6 dioleate, polyglyceryl-2 stearate, PEG-30 dipolyhydroxystearate, sodium stearoyl lactylate, PEG-40 hydrogenated castor oil, hydrogenated palm glycerides, or a mixture thereof.

2. Emulsion according to claim 1, wherein the emulsion is free of silica dimethyl silylate.

3. Emulsion according to claim 1 or 2, wherein the one or more emulsifier(s) other than 1,2-decanediol is/are selected from the group consisting of cetearyl alcohol, glyceryl stearate citrate, polyglyceryl-(3)-methylglucose distearate, potassium cetyl phosphate, hydrogenated palm glycerides, and a mixture thereof.

4. Emulsion according to any one of the preceding claims, wherein the emulsion is a two-phase system of a cosmetic oil-in-water emulsion.

5. Emulsion according to any one of the preceding claims, wherein the emulsion comprises less than 25 wt.-%, preferably less than 20 wt.-%, more preferably less than 10 wt.-%, even more preferably less than 5 wt.-%, most preferably 0 wt.-%, of silicone-containing oils, based on the total weight of the emulsion.

6. Cosmetic emulsion according to any one of the preceding claims, wherein the oil droplets dispersed in the aqueous phase have an average volume-based diameter d_{v0.1} of 1.0 to 10.0 µm, preferably 2.0 to 8.0 µm, more preferably 3.0 to 5.5 µm, as determined by laser diffraction spectroscopy.

7. Cosmetic emulsion according to any one of the preceding claims, wherein the oil droplets dispersed in the aqueous phase have an average volume-based diameter d_{v0.9} of 1.0 to 30.0 µm, preferably 5.0 to 25.0 µm, more preferably 10.0 to 22.0 µm, even more preferably 10.0 to 16.0 µm, as determined by laser diffraction spectroscopy.

8. Use of 1,2-decanediol as a co-emulsifier for reducing the total emulsifier amount in an emulsion according to any one of claims 1 to 7.

9. A process for preparing a cosmetic emulsion according to any one of claims 1 to 7, consisting of or comprising the following steps:
a) providing an aqueous phase;
b) providing an oil phase;
c) combining the aqueous phase and the oil phase,
wherein the aqueous phase and/or the oil phase comprises 1,2-decanediol,
and
wherein the aqueous phase and/or the oil phase is/are heated prior to step c), preferably to a temperature of about 50 to 100°C, preferably wherein the aqueous phase is heated to a temperature in the range of about 70 to 90°C, more preferably to about 80°C, and the oil phase is heated to a temperature in the range of 80 to 100°C, preferably to about 90°C,
and
wherein the combined phases are homogenized after step c) by means of a toothed rim dispersing unit.

## Revendications

1. Émulsion cosmétique comprenant:
une phase aqueuse continue,
des gouttelettes d'huile réparties au sein de la phase aqueuse continue, et
du 1,2-décanediol,
dans laquelle les gouttelettes d'huile réparties au sein de la phase aqueuse continue présentent un diamètre moyen pondéré en volume d_{v 0,5} de 1,0 à 10,0 µm, de préférence de 3,0 à 10,0 µm, encore de préférence de 5,0 à 10,0 µm,
et
dans laquelle l'émulsion comprend en outre un ou plusieurs émulsifiant(s) différent(s) du 1,2-décanediol,
et
dans laquelle ledit un ou lesdits plusieurs émulsifiant(s) différent(s) du 1,2-décanediol sont présents dans l'émulsion en une quantité totale de 0,1 à 5,0 % en poids, de préférence de 0,1 à 3,0 % en poids, encore de préférence de 0,5 à 2,5 % en poids, encore plus préférentiellement de 1,0 à 2,3 % en poids, encore plus préférentiellement de 1,0 à 1,8 % en poids, de manière la plus préférée de 1,6 à 1,8 % en poids, par rapport au poids total de l'émulsion,
et
dans laquelle le 1,2-décanediol est présent dans l'émulsion en une quantité allant de 0,005 à 0,1 % en poids, de préférence de 0,005 à 0,05 % en poids ou de 0,05 à 0,1 % en poids, par rapport au poids total de l'émulsion,
et
dans laquelle ledit un ou lesdits plusieurs émulsifiant(s) différent(s) du 1,2-décanediol est/sont choisi(s) dans le groupe constitué par le stéarate de PEG-100, le cétéaryl glucoside, le chlorure de distéaryldimonium, le chlorure de palmitamidopropyltrimonium, le citrate de stéarate de glycéryle, le citrate d'oléate de glycéryle, le distéarate de méthylglucose de polyglycéryle-3, l'alcool cétéarylique, le phosphate de cétyle de potassium, le phosphate de cétyle de sodium, le polymère croisé d'acrylates/acrylate d'alkyle en C10-30, le polymère croisé d'acryloyldiméthyltaurate d'ammonium/méthacrylate de béhéneth-25, le caprate de polyglycéryl-4, le caprylate/caprate de polyglycéryl-4, le cétyle PEG/PPG-10/1 diméthicone, le dioléate de polyglycéryl-6, le stéarate de polyglycéryl-2, le dipolyhydroxystéarate de PEG-30, le stéaroyl lactylate de sodium, l'huile de ricin hydrogénée PEG-40, les glycérides de palme hydrogénés, ou un mélange de ceux-ci.

2. Émulsion selon la revendication 1, dans laquelle l'émulsion est exempte de silylate de diméthyle de silice.

3. Émulsion selon la revendication 1 ou 2, dans laquelle ledit un ou lesdits plusieurs émulsifiant(s) différent(s) du 1,2-décanediol est/sont choisi(s) dans le groupe constitué par l'alcool cétéarylique, le citrate de stéarate de glycéryle, le distéarate de méthylglucose de polyglycéryl-3, le phosphate de cétyle de potassium, les glycérides de palme hydrogénés, et un mélange de ceux-ci.

4. Émulsion selon l'une quelconque des revendications précédentes, dans laquelle l'émulsion est un système biphasique constitué d'une émulsion cosmétique huile-dans-eau.

5. Émulsion selon l'une quelconque des revendications précédentes, dans laquelle l'émulsion comprend moins de 25 % en poids, de préférence moins de 20 % en poids, encore de préférence moins de 10 % en poids, de manière particulièrement préférée moins de 5 % en poids, de manière la plus préférée 0 % en poids d'huiles contenant des silicones, par rapport au poids total de l'émulsion.

6. Émulsion cosmétique selon l'une des revendications précédentes, dans laquelle les gouttelettes d'huile réparties au sein de la phase aqueuse présentent un diamètre moyen pondéré en volume d_{v 0,1} compris entre 1,0 et 10,0 µm, de préférence entre 2,0 et 8,0 µm, encore de préférence entre 3,0 et 5,5 µm, déterminé par spectroscopie par diffraction laser.

7. Émulsion cosmétique selon l'une quelconque des revendications précédentes, dans laquelle les gouttelettes d'huile réparties au sein de la phase aqueuse présentent un diamètre moyen pondéré en volume d_{v 0,9} de 1,0 à 30,0 µm, de préférence de 5,0 à 25,0 µm, encore de préférence de 10,0 à 22,0 µm, de manière la plus préférée de 10,0 à 16,0 µm, déterminé par spectroscopie par diffraction laser.

8. Utilisation du 1,2-décanediol en tant que co-émulsifiant afin de réduire la quantité totale d'émulsifiant dans une émulsion selon l'une quelconque des revendications 1 à 7.

9. Procédé de préparation d'une émulsion cosmétique selon l'une quelconque des revendications 1 à 7, consistant en ou comprenant les étapes suivantes:
a) fournir une phase aqueuse;
b) fournir une phase huileuse;
c) combiner la phase aqueuse et la phase huileuse,
dans lequel la phase aqueuse et/ou la phase huileuse comprend du 1,2-décanediol,
et
dans lequel la phase aqueuse et/ou la phase huileuse est/sont chauffée(s) avant l'étape c), de préférence à une température comprise entre 50 et 100 °C, de préférence dans lequel la phase aqueuse est chauffée à une température comprise dans la plage allant de 70 à 90 °C, de préférence à environ 80 °C, et la phase huileuse est chauffée à une température comprise dans la plage allant de 80 à 100 °C, de préférence à environ 90 °C,
et
dans lequel les phases combinées sont homogénéisées après l'étape c) à l'aide d'une unité de dispersion à couronne dentée.
